(19)

Europäisches
Patentamt
European
Patent Office
Office européen
des brevets

(11) **EP 4 249 013 A1**

(12) **EUROPEAN PATENT APPLICATION**

(43) Date of publication:
**27.09.2023 Bulletin 2023/39**

(21) Application number: **22163353.0**

(22) Date of filing: **21.03.2022**

(51) International Patent Classification (IPC):
**A61M 5/142** (2006.01)   **A61M 5/145** (2006.01)
**A61M 5/168** (2006.01)   **A61M 5/31** (2006.01)

(52) Cooperative Patent Classification (CPC):
**A61M 5/14248; A61M 5/1452; A61M 5/16831;
A61M 5/3146;** A61M 2005/3143; A61M 2205/3365

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicant: **Ypsomed AG
3401 Burgdorf (CH)**

(72) Inventors:
• **Rytz, Bernhard**
  **3436 Zollbrück (CH)**
• **Burren, Stefan**
  **3150 Schwarzenburg (CH)**

(74) Representative: **Dirix, Yvo
   Ypsomed AG
   Patents
   Brunnmattstrasse 6
   3401 Burgdorf (CH)**

(54) **AN IMPROVED CONVEYING DEVICE FOR COLD INJECTION**

(57)   A conveying device for a medicament delivery device comprising a piston rod which can be advanced along a thrust axis from an initial position to a final position defining a thrust range. A detector configured to detect when the piston rod has reached the final position and a conveying mechanism configured to advance the piston rod along the thrust axis. A control unit configured to control the advancement speed of the piston rod at a target speed wherein the thrust range can be divided in at least two zones:
- a final zone whereby the control unit decreases the target speed to zero once the detector detects that the piston rod has reached the final position, and
- whereby the target speed is decreased to a reduced speed if the detector is activated in a priming zone of the thrust range.

Fig. 6

EP 4 249 013 A1

**Description**

TECHNICAL FIELD

The current invention relates to a conveying device for a medicament delivery device which may be operated at lower temperatures for cold injection.

BACKGROUND OF THE INVENTION

Injection or infusion devices are used for the subcutaneous delivery of liquid medicaments to a patient. The injection or infusion device is either disposable and used once or the device is reusable and can be used multiple times. In the latter case a reservoir containing the medicament can be exchanged for a full reservoir once a previous reservoir has been emptied. For a disposable device, the reservoir may be present in the device (prefilled and ready to use) or the user inserts a reservoir into the single use device. Injection or infusion devices have a conveying device for conveying the liquid medicament from the reservoir into the patient. The conveying device comprises a conveying mechanism powered by an input power package and the input power may be provided by the patient or health care professional for a manually driven injection pen. Alternatively the power package may be electrically, pneumatically, hydraulically or spring driven. A spring driven device may have a pretensioned spring or a user must tension the spring prior to use. The conveying mechanism is designed to transmit a certain amount of power from the input power package via the conveying mechanism to the reservoir for medicament delivery. The force required to expel medicament from the reservoir depends on, for example but not limited to, the dimensions of the reservoir, the length of the fluid path, the dimensions of the needle (needle gauge), the dimensions of a plunger that is advanced within the reservoir, the lubrication of the plunger in the reservoir, the speed of delivery and the viscosity, the rheological characteristics and temperature of the medicament. A theoretical model describing the relationship between the viscosity, needle gauge and length and pressure difference across a needle is the Hagen Poiseuille rule which will described below.

The conveying device is designed to deliver a certain amount of output power from the input power package to the reservoir and can be optimized in terms of transmission ratios and frictional losses and operates in a working range. The working range is thus restricted by an upper force/speed level that can be achieved. If the viscosity of the medicament is too high then the force required for medicament delivery may be out of the working range and the medicament may not be deliverable at the intended delivery rate.

The medicaments are contained in a reservoir, preferably a cartridge or a prefilled syringe. A cartridge comprises a barrel made from glass or a polymer closed by a pierceable septum on one end, and a moveable plunger on the opposite end. The inside surfaces of the barrel, septum and plunger form a volume for the medicament and the septum can be pierced by a hollow needle for establishing a connection to the fluid path. Movement of the plunger, for example by a plunger rod that is part of the conveying mechanism will expel medicament from the cartridge through the fluid path. A prefilled syringe comprises a barrel made from glass or a polymer which may be connected to a needle on one end and comprising a moveable plunger on the opposite end. The needle may be a stacked needle or connected to the syringe via a luer lock connection. Movement of the plunger, for example by a plunger rod will expel medicament from the syringe.

Medicaments in fluid form are based on formulations containing the active ingredient and stabilizers for a long-term stability. The long-term stability also includes the long-term stability of the physical properties such as the viscosity of the formulation. Most medicaments are transported using a cold-chain and are subsequently stored in a refrigerator to ensure long term stability prior to use for a long expiry date of the medicament. This also implies that the viscosity of the medicament may be higher during storage due to the lower temperature. Medicaments, reservoirs containing the medicaments and delivery devices containing the reservoirs, therefore need to be equilibrated to room temperature prior to use as the delivery device is designed for delivery at room temperature. The time needed for tempering the device depends, for example, on the storage temperature, the ambient temperature, the volume of the medicament, the material for the reservoir and the extend of encapsulation of the reservoir within the device. Tempering times may therefore vary between 0.1 and 2 hours, preferably below 1 hour.

If the device is taken out of the cold storage conditions without tempering to room temperature then this will have an influence on the viscosity of the medicament which will be higher and therewith the force required for expelling the medicament from the reservoir may be outside of the working range of the device for delivery at the desired delivery rate. This may result in that the medicament cannot be delivered from the device.

Additionally, delivery of cold medicament to the patient may lead to discomfort due to the combination of lower temperatures and higher viscosities.

Furthermore, certain medicaments when delivered at a high delivery rate may lead to discomfort, for example headaches, to the patient.

In EP3539592A1, a wearable bolus injector (WBI) is disclosed which can be attached to the skin of the patient for medicament delivery. The medicament is contained in a cartridge and delivered by advancing a plunger in the cartridge using a segmented and curved piston rod that is part of a conveying mechanism. The conveying mechanism comprises an electric motor which drives the segmented piston rod using a gearing mechanism. The gear ratios of the gearing mechanism between the electromotor and the piston rod may be adapted to accommodate different viscosities of the medicament in the cartridge unit and/or different needle gauges for the skin needle. The gearing mechanism is thus designed for an intended viscosity of the medicament and cannot accommodate for higher viscosities due to cold injection as this would require an exchange of the gearing prior to delivery. Additionally, the wearable bolus injector has no precautions for temporarily pausing delivery due to patient discomfort as a result of a low injection temperature or due too side reactions related to the active ingredient.

It is an object of the present of the present invention to provide an improved conveying device for a medicament delivery device overcoming the above mentioned drawbacks for cold injection and/or discomfort during delivery. This objective is solved by the independent claim and specific variants are disclosed in the dependent claims.

DEFINITIONS

The term "medicament" or "medication" includes any flowable medical formulation suitable for controlled administration through a means such as, for example, a cannula or a hollow needle and comprises a liquid, a solution, a gel or a fine suspension containing one or more medical active ingredients. A medicament can be a composition comprising a single active ingredient or a pre-mixed or co-formulated composition with more than one active ingredient present in a single container. Medication includes drugs such as peptides (e.g., insulin, insulin-containing drugs, GLP-1 containing drugs or derived or analogous preparations), proteins and hormones, active ingredients derived from -or harvested by- biological sources, active ingredients based on hormones or genes, nutritional formulations, enzymes and other substances in both solid (suspended) or liquid form but also polysaccharides, vaccines, DNA, RNA, oligonucleotides, antibodies or parts of antibodies but also appropriate basic, auxiliary and carrier substances.

The distal end or distal direction is defined by the direction of the needle configured to penetrate the skin of the patient. For an injection pen this may be the injection needle and the end of the pen holding the needle or being configured to hold the needle is the distal end. For an infusion device the distal end and the distal direction is towards the needle configured to penetrate the skin of the patient, which may be along the axis of the device or tilted or perpendicular to the axis of the device. The distal direction in an infusion device represents the direction in which the medicament flows towards the insertion needle. The proximal direction or end is opposite to the distal direction or end.

In the claims, the word "comprising" does not exclude other elements or steps, and the indefinite article "a" or "an" does not exclude a plurality. For example "a detector" does not exclude the fact that there may be two detectors that functionally or structurally fulfill the purpose of "a detector". The mere fact that certain elements or steps are recited in distinct claims shall not preclude the existence of further meaningful combinations of these elements or steps.

GENERAL DESCRIPTION OF THE INVENTION

The current invention relates to a conveying device for a medicament delivery device comprising a piston rod which can be advanced along a thrust axis from an initial position to a final position thereby defining a thrust range for the piston rod. The conveying device further comprises a detector configured to detect when the piston rod has reached the final position and a conveying mechanism configured to advance the piston rod along the thrust axis. A control unit is configured to control the advancement speed of the piston rod at a target speed and the thrust range can be divided in at least two zones:

- a final zone whereby the control unit decreases the target speed to zero once the detector detects that the piston rod has reached the final position, and
- a priming zone whereby the target speed is decreased to a reduced speed if the detector is activated in this initial priming zone of the thrust range.

The piston rod for the conveying device may be a rod with or without threads and the piston rod may be a hollow or a solid (closed) piston rod. The piston rod may be rigid in that it does not bent during the intended use or it is a piston rod that is intended to be bent along a curve for example along a U-shape. The piston rod may be a multi-segment piston rod whereby the segments are connected to each other via, for example, living hinges such that the piston rod can be bent while still being capable of transferring an axial load. The piston rod may also be in the form of a coil spring or spheres connected to each other. A U-shaped piston rod may require adequate guiding means for guiding the piston rod from the linear to the curved shape and back to the linear shape. The guiding means may be provided by a housing or a housing part of the conveying device. The piston rod is preferably intended to transmit an axial load to a plunger present in a reservoir while moving along the thrust axis. The advancement of the piston rod along the thrust axis may be a pure linear advancement or a U-shaped advancement.

The piston rod may push on a plunger in a reservoir from an initial position for a full reservoir, to a final position for an empty reservoir once the medicament has been expelled. When the reservoir is empty, the plunger in the reservoir may be prevented from further movement. The thrust range for the piston rod may thus be defined by the plunger position in a full and an empty reservoir, respectively.

The conveying mechanism may be a pure mechanical conveying mechanism using a power package for advancing the piston rod. Such power packages may be based on mechanical springs, hydraulic or pneumatic sources or gas generators. The mechanical springs may be based on torsional springs or compression springs, using single or multiple springs arranged in parallel or in a series arrangement. Preferably, an electric power package is used for driving the conveying device. The conveying mechanism may have a gearing mechanism to gear up or down a rotation initiated by the power package. As an example, the conveying mechanism may be based on a nut-bolt arrangement between a nut having an internal threading engaging an outside threading of the piston rod. Rotation of the nut with respect to a non-rotating piston rod will advance the piston rod in the conveying direction from the initial position to the final position. This is the arrangement for a solid non-rotating piston rod. Alternatively, the piston rod is rotated by the conveying mechanism and is threadedly engaged with a housing or housing part of the medicament delivery device or with a housing that is part of the conveying device. Rotation of the piston rod will advance the piston rod along the thrust axis. As a preferred alternative, the piston rod is at least partially hollow having an internal threading engaging an external threading of a driver (threaded rod) that is part of the conveying mechanism. Rotation of the driver by the conveying mechanism will advance the at least partially hollow piston rod without rotation.

The advancement of the piston rod may be in a single step for a bolus injector (full advancement along the thrust range at once) or there may be a staged advancement in a plurality of advancement steps. Between each advancement step there may be a time interval where the advancement speed for the piston rod is set at zero by the control unit. For each advancement step the control unit may select a different advancement speed for the piston rod, for example the advancement speed is set at a predefined target advancement speed or at the reduced speed. The target speed may be selected from a set of predefined target speeds and /or the reduced speed may be selected from a set of predefined reduced speeds. The selection may be done based on input parameters from a health care professional or from the selection of the specific medicament or may be based on data received from a sensor such as, for example, a temperature sensor or a flow sensor. The selection may be done by the control unit receiving input data from the sensors or data received from a remote device such as a control device that may be in wireless connection with the medicament delivery device. The selection may also be done based on an internal timer which is started, for example, by removal of the medicament delivery device from its packaging or by the removal of a release liner from a patch device or by pressing or actuating a starting button on the device or on a remote device wirelessly connected to the medicament delivery device.

The control unit may be a pure mechanical controller based on a braking, delay or link-motion mechanism. More preferably, the control unit is an electronic control unit that may be part of and located on a printed circuit board (PCB) and controls an electric component for controlling the advancement speed of the piston rod. The control unit preferably includes a memory or storage unit for storing predefined data such as target speeds, reduced speeds or waiting times or accumulated maximum time values or time limits. The control unit furthermore may comprise an integrated circuit.

The detector is used for multiple purposes; the detector is configured to detect the final position of the piston rod once the piston rod has advanced into the final zone of the thrust range and has reached that final position. Preferably, the final position corresponds to a position where the piston rod is prevented from further movement and consequently, the conveying mechanism cannot advance the piston rod. Once the piston rod is stalled it is important to switch-off the conveying mechanism to prevent damage to the conveying mechanism for the medicament delivery device. The final position may depend on dimensional tolerances of the reservoir (for example the neck of a glass cartridge) and thus may vary between devices. Once the detector has detected the final position within the final zone of the thrust range, the control unit reduces the target speed to zero, e.g. switches the conveying mechanism preferably permanently into an off-state. Preferably, the control unit is programmed such that the conveying mechanism cannot be revived in that the advancement speed of the piston rod can be increased to a level different from zero for a disposable medicament delivery device. For a reusable medicament delivery device, the piston rod for the conveying mechanism can be reset from the final position to the initial position and a new medicament reservoir can be inserted. The control unit can be reset such that the conveying mechanism is configured to advance the piston rod again at the target speed for a new reservoir after the empty reservoir has been exchanged.

If the piston rod is within the priming zone, or the initial part of the thrust range the same detector that is used for detecting the final position within the final zone is used for detecting a stall of the piston rod. If the detector is activated in the priming zone and detects none movement of the piston rod than the detector sends a signal to the control unit and the target speed is decreased to the reduced target speed. The conveying mechanism initially sets the advancement speed for the piston rod at the target speed which is predefined by a target rotational speed for the rotor of the electric motor. The target rotational speed is preferably stored in the storage unit. The conveying mechanism is designed for an operating range of advancement forces and advancement speeds that can be achieved by the conveying mechanism and if the conveying mechanism is not capable of rotating the electric motor at the target rotational speed, for example due to a higher viscosity of the medicament, then the detector detects this non-rotation within the priming zone. The higher viscosity may be due to the lower temperature of the medicament which is stored at lower temperatures in a refrigerator and the medicament is inserted into the delivery device without tempering the medicament to room temperature. Alternatively, the prefilled medicament delivery device including the medicament reservoir is taken out of the refrigerator and used without tempering the prefilled device to room temperature. The viscosity of the cold medicament will be higher thus leading to higher forces required for expelling medicament from the delivery device. The force is lowered that is required for advancing the piston rod at the reduced target speed and expelling the cold medicament with a higher viscosity (Hagen-Poiseuille Law) such that the cold medicament will be expelled at the reduced delivery rate. Optionally, the controller is programmed to further reduce the advancement speed of the piston rod further to a second reduced target speed if the detector is still activated and detects non advancement at the reduced target speed within the priming zone. This process may be repeated until a final reduced target speed was not successful in advancing the piston rod at the final reduced target speed and the control unit may signal an alarm to the user. The advantage of decreasing the target speed to a reduced target speed or further decreasing the target speed to a second, third etc. reduced target speed is that the medicament can be expelled from the medicament delivery device even if the instructions for tempering the device are ignored or if the device is taken from the refrigerator in an emergency situation.

There may be a transition time between the switching from the target advancement speed to the reduced speed. The transition time, or holding time means that temporarily the advancement speed is automatically set to zero by the control unit and after a predefined transition or holding time, the control unit automatically sets the advancement speed from zero to the reduced target speed. The holding or transition time before switching to the second or third reduced target speed may be longer compared to the holding /transition time before the first reduced target speed. The conveying mechanism may comprise a gearing mechanism which is tensioned when the conveying mechanism initially attempts but fails to advance the piston rod at the target speed. The transition time before switching to the reduced target speed intends to reduce the stresses build up in the conveying mechanism. This prevents damage to the conveying device and therewith increases the reliability. Additionally, the holding/waiting time implies that the medicament temperature increases such that the viscosity is lowered and the device can be operated at the reduced target speed. This also increases the reliability of the device when it is directly taken from the refrigerator without warm-up.

In an example, the priming zone of the conveying device is defined by the initial part of the thrust range and the final zone is defined by the final part of the thrust range. The priming zone is defined by that less than 30% of the initial part of the thrust range has been reached by the piston rod and the final zone is starting above 80% of the final part of the thrust range.

The thrust range can be subdivided in at least the priming zone for the initial part of the thrust range and the final zone for the final part of the thrust range. The priming zone is defined by less than 30% of the initial part of the thrust range, preferably less than 20% of the initial part of the thrust range, more preferably less than 10% of the thrust range, most preferably less than 5% of the thrust range. The final zone starts above 80% of the final part of the thrust range more preferably above 90% of the thrust range, most preferably above 95% of the thrust range. Between the priming zone and the final zone there may be other zones within the thrust range, for example an occlusion zone where, when the detector is activated, an alarm signal is generated and directed to an acoustic or optical signaling unit. The alarm signal may indicate that the medicament cannot be delivered due to an occlusion within a flow path of the medicament. Dividing the thrust range in several zones has the advantage that in each zone a specific response can be initiated by the control unit still using the signals from the same detector.

In another example, the target speed of the conveying device is stepwise or continuously decreased to zero or to the reduced speed. A continuous reduction may be beneficial in gradual reduction of mechanical stresses that were built up in the conveying mechanism attempting to advance the piston rod at the target speed.

The reduced speed for the conveying device may be below 60% of the target speed. The reduced speed may be below 60% of the target speed, preferably below 50% of the target speed, most preferably below 40% of the target speed. The second reduced speed may be below 50% of the target speed, preferably below 40% of the target speed most preferably below 30% of the target speed. The third reduced speed may be below 40% of the target speed, preferably below 30% of the target speed, most preferably below 20% of the target speed. Reduction of the target speed will increase the injection time for delivery the amount of medicament present in the medicament delivery device.

In yet another example, the target speed in the priming zone is first reduced to zero before being set at the reduced speed. The reduction to zero speed initiates the transition time or holding time for stress relaxation in the conveying mechanism mentioned above.

In another example, the piston rod of the conveying device may be advanced using an electric drive comprising an electric motor and the detector may be activated by detecting non-rotation or limited rotation of the electric motor. The conveying mechanism of the conveying device may use an electric motor for advancing the piston rod. The electric motor is powered by a battery or accumulator and rotation of the rotor of the electric motor is used for advancing the piston rod. If the piston rod cannot be advanced, for example in the final zone where the reservoir has been emptied and the piston rod has reached its final position, or in the priming zone where the cartridge is full but cannot be advanced due to an increased viscosity of a medicament, then the non-advancement of the piston rod will, due to its mechanical engagement with the conveying mechanism, result in non-rotation or limited rotation of the electric motor thereby activating the detector. Limited rotation is defined that less than 70% of the intended rotational speed (revolutions/second) can be reached, preferably less than 50% most preferably less than 30% of the intended rotational speed can be reached.

The detector preferably detects or is based on detecting rotation or non-rotation of the electric motor by measuring the steps of the electric motor, or measuring the back-emf signal, or using an encoder, or measuring the motor current. Measuring the steps of an electric motor and step losses indicates that the electric motor cannot be driven as intended therewith initiating a detector signal to the control unit. The control unit may compare the steps or accumulated steps with a stored value using a comparator and detects non- rotation if there is a deviation. Alternatively, the back-emf signal generated by the electric motor is used for detecting rotation or non-rotation of the electric motor. In yet another alternative an encoder, for example a magnetic or an optical encoder is used to detect rotation or non-rotation of the electric motor.

In yet another example, the control unit activates the electric motor of the conveying device for advancing the piston rod at the target speed by rotation of the electric motor at a target rotational speed and the control unit decreases the target rotational speed of the electric motor to zero when the final position for the piston rod has been reached and is detected by the detector, or decreases the target rotational speed to a reduced rotational speed for advancing the plunger at the reduced speed when the detector is activated in the priming zone. The target rotational speed for the electric motor is transmitted to the piston rod using the conveying mechanism that may comprise a gearing mechanism and the rotational target speed is thereby converted into a linear advancement speed for the piston rod.

In another example, the control unit of the conveying device may receive information from the detector and measure and count the number of rotations or steps of the electric motor and compare the accumulated number of rotations or steps with stored values. If the accumulated value is below a stored priming value when non-rotation is detected then the target rotational speed is decreased to the reduced rotational speed and if the accumulated value is above a stored upper value then the target rotational speed is reduced to zero. The priming zone is thus defined by the range between zero revolutions and the priming value whereas the final zone starts by the upper value for the accumulated number of revolutions. The stored values are stored within the storage unit and if the accumulated values representing the number of rotations of the electric motor are below a priming value then the piston rod is still within the priming zone of the thrust range. As long as the piston rod is within the priming zone and the accumulated number of rotations is below the priming value, the target rotational speed for the electric motor is decreased to the reduced rotational speed. Optionally, there is the transition time or holding time between the detection of non-rotation in the priming zone and setting the rotational speed to the reduced rotational speed.

As a further example the control unit may activate a first optical and/or acoustic signal when the piston rod is advanced at the target speed and may activate a second optical and/or acoustic signal different from the first signal when the piston rod is advanced at the reduced target speed and may activate a third optical and/or acoustic signal different from the first and second signals when the piston rod has reached the final position. The optical signal may be based on a pure mechanical indicator or a mechanical components may be rotated by the electric motor. For example an optical indicator may be mechanically moved into, and visible in a window in the housing of the medicament delivery device. Alternatively and preferably, the optical indicator is electrical and based on a light source such as a LED indicator visible in a viewing window or through a translucent part of the housing. There may be a plurality of optical indicators, for example a plurality of LED lights each having the same color or a different color. The plurality of LED lights may form a pattern and may indicate, for example by a blinking frequency that the device is operated at the intended target speed for the piston rod. Additionally, the LED indicators may be oriented in an array and are activated sequentially depending on the amount of medicament delivered from the device thereby forming a status indicator. The acoustic signal may be based on a buzzer or loud speaker that is part of the control unit. Optionally, the first optical signal is signaled in a first color whereas the second and third optical signals are signaled in a different color. Optionally, the first acoustic signal may be at a different frequency compared to the second or third acoustic signals.

In another example the target rotational speed for the conveying device may be decreased to zero when the accumulated number of rotations is between the priming value and the upper value and the control unit activates a fourth acoustic and/or optical alarm signal indicating an occlusion occurring in an occlusion zone located between the priming zone and the final zone. Activation of the detector in a zone of the thrust range between the priming zone and the final zone may be indicative of a failure during delivery, for example an occlusion within the fluid flow path of the medicament therewith increasing the safe operation of the device for the user.

Optionally, the conveying device may comprise a pausing button which pauses medicament delivery when pressed by setting the target rotational speed of the electric motor temporarily to zero. All above mentioned examples for conveying devices comprising features for decreasing the advancement speed for the piston rod to a reduced speed in the priming zone, or decreasing the advancement speed to zero in the final zone, or the introduction of the transition/holding times during the priming zone are all controlled by the control unit and comprise automatic or programmed steps executed by the device upon activation of the medicament delivery device by an user or a health care professional. The pausing button is intended to temporarily pause medicament delivery and sets the device in a pausing mode. The pausing button is pressed or actuated by the user or by a health care professional after starting delivery, e.g. after activation of the device by, for example, pressing a start or actuation button. Alternatively, actuations is started using a capacitive sensor acting as the starting button. The pausing button may thus overrule the steps initiated by the control unit after starting injection. The control unit may continue with medicament delivery after the pausing button has been pressed a second time. The time interval for the pausing mode between the first and second activation of the pausing button defines a pausing time. There may be a maximum pausing time stored in the storage unit of the control unit and once the pausing time exceeds the maximum pausing time then the conveying device automatically returns to advancing the piston rod or, alternatively, aborts the injection. The pausing button may be pressed more than once thereby setting the device again in the pausing mode. There may be no limit set to the number of times the pausing button can be pressed for actuating the pausing mode. Alternatively, the number of times for activating the pausing mode may be limited to four times, preferably three times, most preferably two times. The maximum pausing time for the accumulated pauses is restricted to one hour, preferably thirty minutes, more preferably to twenty minutes. The pausing button may be a button that can be pressed on the medicament delivery device or the pausing mode is initiated via a remote device wirelessly connected to the medicament delivery device. The medicament delivery device may have a timer included in the control unit counting the time required for delivery at the target speed, at the reduced speed, counting the holding times and counting the time that the device is operated in the pausing mode. The accumulated time value may be compared with a maximum time value for delivery of the medicament and a time-out signal or alarm signal may be initiated once the maximum time value has been reached. The maximum time value may be four hours, preferably three hours, more preferably two hours most preferably one hour.

Optionally, the pausing mode is accompanied by a fifth acoustic and/or optical signal. The fifth acoustic signal may be a melody indicating the start of the pausing mode and the same melody is played indicating the end of the pausing mode for resumption of medicament delivery. The melody at the start and the end of the pausing mode may be reversed. Alternatively, an acoustic buzzer signal is activated at the start of the pausing mode different from the signal at the end. The difference may be a different buzzing frequency or different noise level. The pausing mode may be accompanied by a specific pausing melody that is different from a melody used for delivery at the target speed or from a melody used for delivery at the reduced target speed. The pausing function may be enabled or disabled for the medicament delivery device. The pausing function may be enabled as a default on the device and disabled either by pressing the pausing button for a longer period of time, for example at least three second, preferably at least five second more preferably at least ten seconds. The pausing function may also be enabled or disabled using a remote device comprising a control application for the medicament delivery device.

The pausing button for activating the pausing mode may be the same button as the actuation button used for starting medicament delivery. The button may be switched automatically and irreversibly from an actuation button function to a pausing button function after starting medicament delivery by pressing the actuation button once. Alternatively, the actuation button may be pressed for a longer period of time and thereby switches from the actuation button function to the pausing button function. The pausing button may automatically return to the functionality of the actuation button or it may require another prolonged pressing of the pausing button for returning to the actuation button function. Medicament delivery of a medicament with a high viscosity and/or lower temperature (cold injection) may lead to discomfort by the patient and therefore the patient can delay medicament delivery using the pausing button. Additionally, certain medicaments may lead to local pain or headaches when delivered at once requiring a pausing button.

A medicament delivery device comprising the conveying device described above further comprises a reservoir containing a medicament and a plunger moveable within the reservoir by the piston rod for expelling the medicament from the delivery device through an outlet of the reservoir. The medicament delivery device may be an injection or an infusion device, it may be a reusable or a disposable device. The medicament delivery device may be a patch device such as a patch pump or a wearable bolus injector (WBI). The reservoir is preferably a rigid reservoir made from glass or a plastic material such as a cycloolefinic polymer (COC) or polypropylene (PP). The reservoir may be a prefilled syringe comprising a needle or outlet with a luer lock at the distal end or the reservoir is a cartridge closed by a pierceable septum at the distal end. The cartridge preferably comprises a cylindrical wall section having one opening closed by the septum and an opening opposite to the first opening closed by the moveable plunger. The volume between the wall, the plunger and the septum is available for the medicament. Linear advancement of the plunger rod at the target advancement speed (for example in mm/min) advances the plunger towards the outlet thereby reducing the volume available for the medicament thereby expelling the medicament in terms of a target delivery rate (for example in ml/min). In addition to the above patch device, the medicament delivery device may be a pen-shaped injection device hand-held by the user during injection and devoid of a patch or adhesive layer. The reservoir may be a prefilled syringe comprising a needle and a plunger advanced in dispensing direction by a non-manual drive such as an electric motor powered by a battery.

Specifically, the injection device may be an electronically controlled auto-injector adapted for dispensing the entire volume of the reservoir in a single injection event over a period of time in excess of 30 seconds, preferably in excess of 5 minutes.

The medicament delivery device whereby the initial position of the piston rod is defined by a plunger position for a full reservoir and the final position is defined by a plunger engaging a neck region of the reservoir preventing further plunger advancement (thereby preventing further advancement of the piston rod). The reservoir preferably is a cartridge with a wall forming a barrel for moving the cylindrical plunger having a first diameter adjusted to the inner diameter of the barrel. The barrel may be made of glass or a polymer. The diameter of the barrel is reduced close to the septum forming a neck region to accommodate the outer dimensions of the septum. The plunger is moveable from the initial position close to the proximal end until the neck region as the plunger having the first diameter cannot accommodate or pass the neck region with a lower diameter.

As an example, the medicament delivery device is a wearable bolus injector. The bolus injector is preferably prefilled and ready to use and comprises and adhesive for adhering the device to the skin of a patient.

The medicament delivery device may further comprise a reservoir with a plunger which is moveable within the reservoir at a target speed from an initial position to a final position defining a thrust range thereby expelling medicament from the delivery device. The medicament delivery device further comprises a detector configured to detect when the plunger has reached the final position in the reservoir and a conveying mechanism configured to convey the plunger in the reservoir at the target speed. A control unit of the medicament delivery device is configured to control the target speed and to decrease the target speed to zero when the detector is activated and detects when the plunger has reached the final position. The medicament delivery device is characterized in that the target speed is decreased to a reduced speed if the detector is activated during the priming zone of the device.

In another example, the medicament delivery device comprises a reservoir containing the medicament and a moveable plunger. The conveying mechanism is configured to advance the plunger towards an outlet of a reservoir to expel medicament from the device, preferably using a piston rod. The control unit comprises a control circuitry controlling the conveying mechanism and a start button which when pushed starts a delivery mode for medicament delivery by activating the conveying mechanism. The medicament delivery device further comprises a pause button, preferably part of the control circuitry, which when pushed pauses medicament delivery by temporarily deactivating or halting the conveying mechanism thereby starting a pausing mode. The delivery of the medicament is resumed once the pause button is pushed for a second time thereby defining a pausing time, wherein the delivery mode is automatically resumed when the pausing time exceeds a maximum pausing time value that is stored in the storage unit. Alternatively, the injection is completely aborted once the pausing time exceeds the maximum value stored. Optionally, the length of the maximum pausing time in the pausing mode may be configured or selected from a range of maximum pausing times and the pausing mode may be activated multiple times. The pausing times may be recorded and accumulated by the control unit and compared to a maximum accumulated pausing time in the storage unit and the delivery mode is automatically activated once the accumulated pausing times exceed the maximum accumulated pausing time. Optionally, the number of times the pausing mode can be activated can be configured by the user and the pausing mode may be enabled or disabled by the user as well.

An acoustic and/or optical pausing signal may be produced by the control unit during the pausing mode that is different from the acoustic and/or optical delivery signal during the delivery mode and an acoustic and/or optical resumption signal is produced when switching from the pausing mode to the delivery mode or vice versa from the delivery mode to the pausing mode that is different from the pausing signal or the delivery signal. For example, the acoustic signal comprises a tone sequence and the tone sequence for the pausing signal and the delivery signal are reversed. Optionally the pause button equals the start button, the start button may be switched to the pause button by prolonged pushing of the start button or the start button is a two-step push button. The advantage of the pausing button is that the pausing mode can be delivered once the patient senses discomfort during medicament delivery.

LEGENDS TO THE FIGURES

While the invention has been described in detail in the drawings below and foregoing general description, such description is to be considered illustrative or exemplary and not restrictive. Variations to the disclosed embodiments can be understood and effected by those skilled in the art and practicing the claimed invention, from a study of the drawings, the disclosure, and the appended claims.

Figure 1: A wearable bolus injector.
Figure 2: Cross section of the wearable bolus injector of Figure 2.
Figure 3: Details for the conveying device and needle insertion device.
Figure 4: Schematic representation of the temperature and the viscosity of a medicament after tempering from cold storage.

Figure 5:     Schematic drawing for delivery at the target speed with detection when the piston rod has reached the final position in the final zone.

Figure 6:     Schematic drawing of the target speed and reduced speed in the priming zone during cold injection.

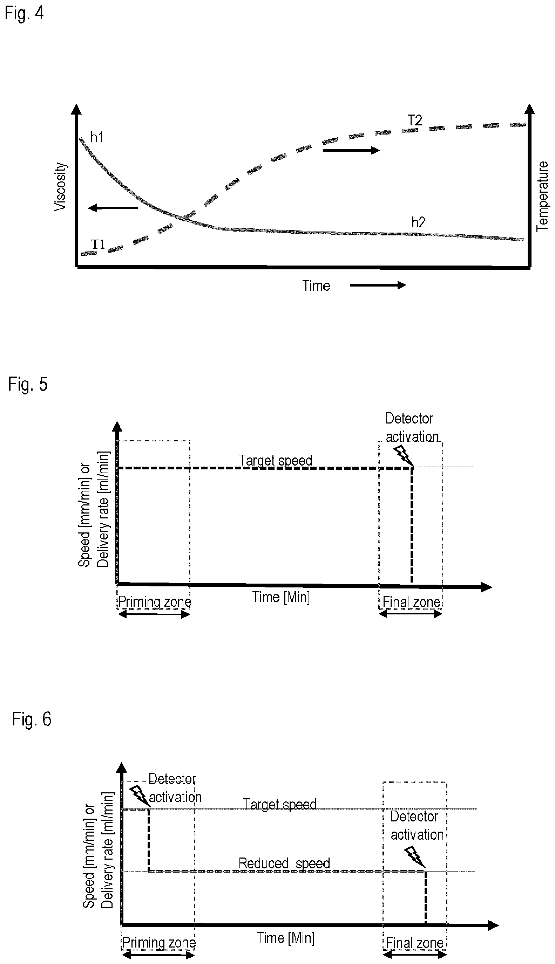

Figure 6:     Schematic drawing for cold injection including a transition time

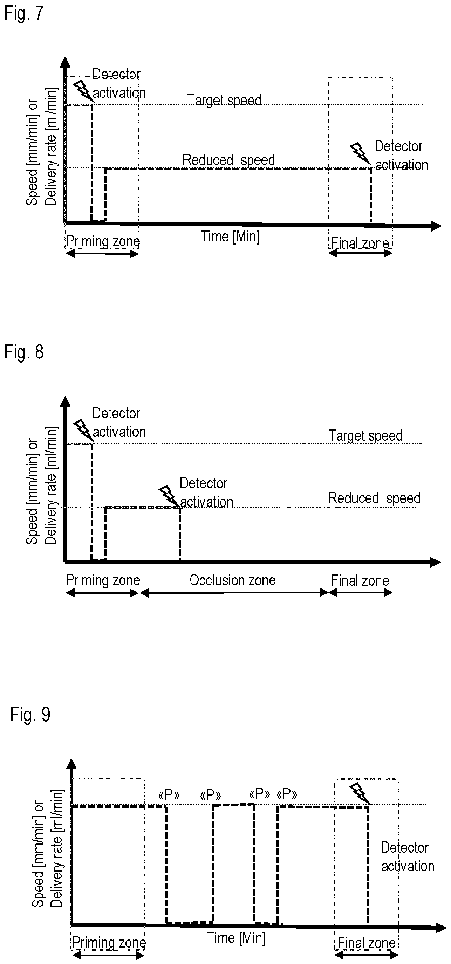

Figure 7:     Schematic drawing for cold injection with transition time and pausing button activated.

Figure 8:     Schematic drawing for cold injection with transition time and occlusion detection.

Figure 9:     Schematic drawing for delivery at the target speed including a pause mode.

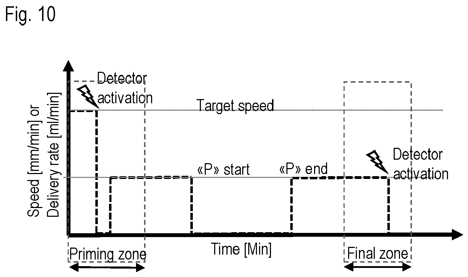

Figure 10    Schematic drawing for cold injection with transition time and pause mode.

DETAILED DESCRIPTION OF THE FIGURES

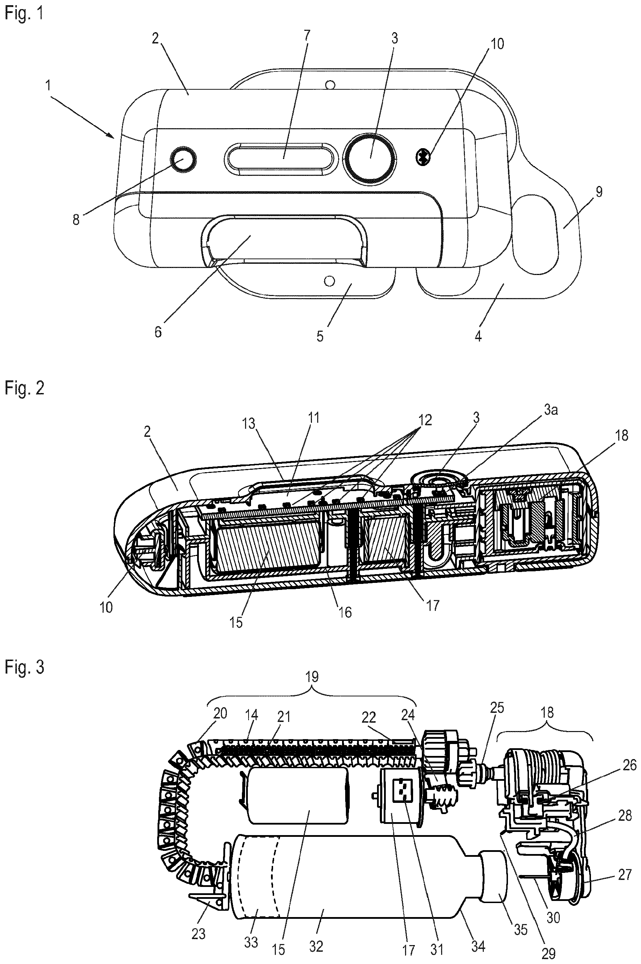

A medicament delivery comprising the conveying device according to the present invention is shown in Figures 1 to 3. The medicament delivery is a wearable bolus injector WBI 1. Alternative medicament delivery devices such as an autopen, an insulin pump, a patch pump, a patch injector or an electric auto injector can also accommodate the conveying device according to the present invention. The WBI 1 comprises a housing 2 enclosing and/or holding the units that make up for the delivery device. The housing 2 may have a viewing window 6 for viewing at least a part of a medicament reservoir. The viewing window 6 may be part of a separate cover that is attached or attachable to the housing 2. The housing may have a status indicator 7. The status indicator may be based on a translucent or transparent part of the housing that is backlit by a light source, for example by a plurality of LED lights. The status indicator 7 may be used to indicate progress of delivery, the delivery speed or whether the device is operated at the target delivery rate/speed or at the reduced delivery rate/speed. The status indicator may also indicate a general status of the device such as "ready to use", "delivery completed" or "ready for skin removal". The WBI may have a second LED indicator 10 which may be used to indicate the connection to an external device, for example indicating that a bluetooth connection has been established or that the connection is lost. Alternatively, the second LED indicator is used to indicate if a connection establishment is possible. Or in other words the device is visible for other devices for connections The second LED indicator may have a different color compared to the status indicator 7. For example the color blue may be used for indicating connectivity to an external device. A different color, for example the color red, may be used for the status indicator 7 and/or second LED indicator 10 to indicate a malfunctioning of the device such as an "occlusion" or "battery empty" or "connection lost".

A start button 3 is accessible to the user for actuation of the WBI. The start button 3 is preferably a push button that may be flat with the housing 2 or may extend from the housing. Alternatively, the button is a pull button. The push or pull button may be a multi-level, for example two level button whereby one push/pull level initiates a delivery mode for medicament delivery whereas a second level can be used for starting different modes such as, for example, a pausing mode, or a final mode for disposal of the device, or a mode for resetting the (reusable) medicament delivery device. Optionally there may be a second push or pull button 8 that is separate from the start button 3. Alternatively, reference number 8 identifies another location for the Bluetooth status indicator. The button 3 and 8 may also be actuation buttons using capacitive sensors (touch sensors). The second push/pull button may also be used for starting the different modes. The WBI is configured for attachment to the skin of a patient using a patch 5 comprising a skin adhesive. The patch 5 has at least a second adhesive layer for attachment to a bottom surface of the housing 2. The patch 5 preferably is a multilayered system comprising a carrier layer and at least the two above mentioned adhesive layers. The carrier layer may be for example a woven layer made from natural cotton fibres or from polymer fibres. The skin adhesive may be based on an acrylic resin. A combination of a normal adhesive and a pressure sensitive adhesive may be used. The skin adhesive layer for the patch 5 is covered by a release liner 4 preventing unintended attachment of the delivery device and protecting the skin adhesive prior to use. The release liner 4 may be based on paper or on a polymeric film (for example a polyester film) and one or both surfaces of the film may be coated with a non-adhesive coating. The release liner 4 may have a pull tab 9 that optionally has an opening to facilitate gripping of the release liner.

The medicament delivery device presented in Figure 1 comprises at least the following units: A reservoir unit comprising a reservoir holder holding a reservoir, a conveying unit for expelling medicament from the reservoir, a patch unit for attaching the device to the skin, a needle insertion unit comprising a fluid path, and a control unit controlling the conveying unit and the needle unit. All units are housed in the housing 2 providing protection from the ambient and mechanical support to the units. The control unit may be configured to communicate with an external device, preferably via a Bluetooth connection. The needle insertion unit comprises at least a hollow skin insertion needle and a skin insertion mechanism. The skin insertion mechanism is configured to move a tip of a skin needle from a position within the housing to a position outside the housing. The housing 2 comprises an aperture for the skin needle that may be covered by a sterile barrier prior to use. The needle unit further comprises a tubing connected to the skin needle directing the fluid from the reservoir through the skin needle. The other end of the tubing may be directly coupled to the reservoir or comprises an additional needle that may move from a position within the needle unit towards the reservoir unit to couple the fluid path of the needle unit to the reservoir in the reservoir unit. The above mentioned fluid path is at least formed by the tubing, the hollow skin insertion needle and optionally the additional hollow needle. The patch unit comprises a multilayered patch with the skin adhesive and housing adhesive and may furthermore comprise a skin contact sensor such as a capacitive sensor. Examples and details for the units are presented below.

The control unit 11 shown in Figure 2 is preferably located on a printed circuit board (PCB) comprising an integrated circuit, a storage unit and LED lights 12 located adjacent to a transparent housing section 13. An array of LED lights 12 may be part of and form the status indicator 7. The start button 3 may activate a switch on the PCB to start or pause medicament delivery. In the present example, the button can be pushed one level for starting the device. Alternatively the push button and PCB switch may allow for different functions depending on the pressure applied or the position of the push button with respect to the housing, or the amount of time pressure is applied. The conveying device comprises a piston rod 14 a battery 15, an electric motor 17 and a gearing mechanism that will be shown in detail in Figure 3. The battery 15 is enclosed in a battery holder and electrical contacts may directly contact the PCB. The electric motor 17 is housed in the conveying unit and may also have electrical contacts 31 contacting the PCB. The PCB may further have a transmitter/receiver for connecting to an external device, and/or the PCB may further comprise a buzzer, and/or the PCB may comprise a loudspeaker for acoustic signaling.

Details for the conveying device 19, the needle unit 18 and the reservoir unit with reservoir 32 are shown in Figure 3. The reservoir unit comprises a cartridge 32 closed on one end by a moveable plunger 33 and by a septum 35 on the opposite end. The septum is preferably connected by a crimp to a neck 34 of the cartridge. The neck 34 has a reduced diameter compared to the cartridge's barrel receiving the moveable plunger. Optionally, the septum is covered by a sterile barrier film that may be removed prior to medicament delivery. The needle unit 18 comprises a skin needle insertion mechanism 26 configured to move a skin needle 29 from a retracted to an extended position. The insertion mechanism is preferably spring driven. The skin needle insertion mechanism may also be configured to retract the skin needle 29 from the extended to the retracted position. The needle unit 18 further comprises a cartridge needle insertion mechanism 27 configured for insertion of a cartridge needle 30 through the septum of the cartridge 32. A tube 28 connects the cartridge needle 30 with the skin needle 29 establishing the fluid connection between the medicament in the reservoir and the patient. The tube and both hollow needles are part of a fluid path. The needle unit 18 is enclosed in a not shown needle unit housing. The needle unit housing comprises at least two passage for the skin needle 29 and the cartridge needle 30 and both passages may be covered by removable membranes. A further passage may be available for a cam shaft as discussed below. Preferably the needle unit 18 is sealed from the ambient and sterilized. The passages may be sealed by a sealing, for example by a sealing ring for the cam shaft 25, or the passages are covered by the removable membranes such as Tyvek membranes. The skin needle and cartridge needle in the needle unit 18 are preferably oriented perpendicular to each other.

The conveying device 19 comprises the segmented piston rod 14 having segments 20, a first segment 22 and a last segment 23. The segments 20 for the segmented piston rod 14 are connected to each other by hinges such that the piston rod can be bent and form a U-shaped piston rod. The piston rod 14 is guided by a not shown housing of the conveying device. The last segment 23 is adapted to directly or indirectly abut the plunger 33 in the cartridge and advancement of the piston rod 14 will advance the plunger for expelling medicament from the device. A cartridge adapter may be provided between the last segment 23 and the plunger 33. The piston rod 14 is advanced by rotation of the threaded rod 21 having an external threading that engages an internal threading of the first segment 22 of the piston rod 14. The piston rod 14 is guided by the housing of the conveying unit such that the piston rod cannot rotate around its own axis while being capable to form the U-shaped configuration due to the hinges between the segments. Rotation of the threaded rod 21 advances the piston rod 14 and thus advances the plunger 33 in the cartridge. The plunger can be advanced from the initial position for an least partially filled cartridge until the plunger has reached the neck 34 of the cartridge, as the neck portion prevents further movement of the plunger 33. The conveying unit 19 further comprises the electric motor 17 and battery 15. The rotation of the electric motor 17 is transmitted via a gearing mechanism 24 to the threaded rod 21.

The electric motor 17 is preferably configured to operate in two rotation directions, a first rotation direction the rotation is transmitted via the gearing to the threaded rod 21 whereas a rotation into the opposite direction is transmitted to a cam shaft 25. The cam shaft 25 is part of the needle unit and rotation of the cam shaft 25 controls the release of the skin needle insertion and the cartridge needle insertion. The gearing 24 may comprise a one-way ratchet system such that only one rotation direction is transmitted to the cam shaft 25 or to the threaded rod 21.

The conveying device comprises a detector for detecting when the piston rod 14 has reached the final position in the cartridge 32. When the plunger 33 advances to the distal end of the cartridge, then the distal end of the plunger contacts the neck 34 of the cartridge 32 and the plunger will be prevented from further movement. The cartridge has been emptied and no further medicament can be expelled. As the plunger 33 cannot be advanced any further also the piston rod 14 is prevented from further movement and this will stall rotation of the threaded rod 21, the gearing 24 and finally also the rotation of the electric motor 17. The rotor of the electric motor 17 may continue rotating for a short period of time when the plunger 33 has already reached its final position due to the tolerances and elasticity of the drive train. This may result in mechanical stress built-up within the conveying device. The electric motor may have a detector for detecting rotation or non-rotation of the electric motor. The detector may include an encoder that is integrated within the electric motor or that is a separate component. The encoder may be an electrical, an optical or a magnetic encoder. An optical encoder may be based on a winged wheel positioned between a light source and an optical detector. The winged wheel will typically be coupled to the output of the electric motor to indicate the rotation. An example of a magnetic encoder is a hall sensor. Alternatively, the detector may also detect electric signals such as a back-emf voltage or measure the motor current for detecting rotation or non-rotation of the electric motor. In this example the detector is based on detecting the back-emf signal.

The detector detects rotation or non-rotation of the electric motor as the piston rod has been stalled. This may be because the plunger has reached the final position after expelling all medicament that can be delivered from the device, or because there is an occlusion within the fluid path (for example within the insertion needle 29 or tube 28) preventing further medicament delivery or because the medicament has been removed from cold conditions without tempering, so called cold injection. In the latter case the viscosity is too high preventing delivery. In all cases, the detector detects non-rotation and sends a signal to the control unit which will send different instructions to the electric motor depending on the position of the piston rod along the thrust range:

- In the final zone of the thrust range the control unit will stop sending instructions to the electric motor to continue or attempt rotation of the rotor of the electromotor as the cartridge has been emptied. The target rotational speed for the rotor will be set to zero. The control unit may activate an acoustic and/or optical signal for successful completion of medicament delivery. Details will be presented and discussed in Figure 5.
- In the initial priming zone of the thrust range, the control unit will reduce the rotational speed of the electric motor to the reduced rotational speed resulting in the reduced advancement speed for the piston rod. The control unit may activate a different signal for signaling, for example, cold injection. Details will be presented and discussed in Figures 6 and 7
- In the occlusion zone between the priming and final zone, the control unit sets the target rotational speed of the electric motor to zero and may activate an alarm signal. Details will be presented in Figure 8

In the following, the fluid flow through a hollow needle, for example the cartridge needle 30 or the skin needle 29 is described according to the Hagen Poiseuille Equation (HPE). The HPE describes the laminar flow of an incompressible fluid with viscosity $\eta$ through a needle with constant diameter (r) and length (1). The pressure drop ($\Delta P$) required for a delivery rate (dV/dt) is given by:

$$\dot{V} = \frac{dV}{dt} = \frac{\pi \cdot r^4}{8 \cdot \eta} \frac{\Delta p}{l} = -\frac{\pi \cdot r^4}{8 \cdot \eta} \frac{\partial p}{\partial z}$$

As the viscosity $\eta$ increases with decreasing temperature of the medicament, also the pressure drop $\Delta P$ needs to increase for a constant delivery rate. Pressure drop $\Delta P$ is the pressure difference at the entrance and exit of the needle. For delivery from a cartridge this is defined by the force applied to the plunger (by the piston rod) divided by the surface area of the plunger (N/mm$^2$). A higher pressure P on the plunger requires a higher force F and there is a maximum force the conveying mechanism is capable to deliver which may lead to blockage of the drive train when the target delivery rate is selected at a certain viscosity.

At constant pressure P (or force F delivered by the conveying mechanism), and constant length 1 the ratio between the target delivery rate ($\eta$1) at and the reduced delivery rate ($\eta$2) can be calculated as:

$$V_{target} * \eta 1 = V_{reduced} * \eta 2$$

A schematic representation for the viscosity ($\eta$) and temperature (T) of a medicament as a function of time is presented in Figure 4. If a medicament is removed from cold conditions, for example from a refrigerator at 5 degrees Celsius then the viscosity ($\eta$1) is higher compared to a tempered medicament at 20 degrees ($\eta$2). Injection without tempering (cold injection) will increase the force required for delivery and may be beyond the operating range of the conveying mechanism. Decreasing the delivery rate by reducing the advancement speed of the piston rod from the target speed ($V_{target}$) to the reduced speed ($V_{reduced}$) allows for medicament delivery without changing the mechanics of conveying device (for example the gearing mechanism) as suggested in the prior art EP3539592.

In Figures 5 to 10, exemplary embodiments for a conveying device or for a medicament delivery device comprising the conveying device that is adapted to cold injection and/or comprises a pausing mode are presented. The conveying device is preferably the conveying device for the WBI as depicted in Figures 1 to 3.

Figure 5 presents a schematic representation for a successful delivery of the medicament at the target speed/delivery rate. The device comprising the medicament and conveying mechanism has been tempered to room temperature and the viscosity is adapted accordingly. The user removes the release liner and attaches the medicament delivery device to the skin and starts delivery by pushing the start button 3. The needle unit 18 is activated and the needles 29, 30 are inserted into the skin and cartridge to establish the fluid connection between the patient and the medicament in the cartridge. The control unit activates the electric motor 17 and the piston rod 14 can be advanced at the target speed whereby the rotor of the electric motor 17 rotates at the target rotational speed. The back-emf signal is detected and sends signals to the control unit and the control unit counts and accumulates the number of rotations. The accumulated number of rotations is compared with a stored upper value and the final zone for the thrust range has been reached with the upper value. Detection of non-rotation in the final zone (as no back-emf signal is available anymore) sets the target rotational speed for the electric motor to zero. The delivery has been successfully completed from the device and the control unit may activate an acoustic or optical signal accordingly. Additionally, the needle unit is activated to retract the skin needle from the patient. Another optical and/or acoustic signal may be provided indicating to the user that the device may be removed from the skin. Optionally, a waiting-time or dwell-time is started between completion of delivery and device removal. This allows the tissue enough time to accommodate or diffuse the medicament within the tissue prior to removal. An acoustic and/or optical signal may be transmitted to the user signaling delivery at the target delivery rate.

Figure 6 shows a schematic representation for cold injection. The device is removed from the refrigerator and used without tempering to room temperature leading to a higher viscosity for the medicament. The control unit 11 initially instructs the conveying device for delivery at the target speed. The viscosity is however too high and prevents delivery at the target speed and the detector is activated within the priming zone or initial zone of the thrust range. Thus the number of accumulated rotations detected is below the priming value when non-rotation is detected as no back-emf signal is received by the control unit. In this example the priming zone is defined as below 10% of the thrust range. The control unit instructs the electric motor for medicament delivery at the reduced speed and the back-emf signal for rotation is received by the control unit which counts and accumulates the number of rotations at the reduced speed. The detector is activated once again when the plunger has reached the final position within the final zone of the thrust range leading to non-rotation of the electromotor and the reduced target speed is further reduced to zero. An acoustic/and or optical signal may be transmitted to the user signaling delivery at the reduced speed.

Another example for cold injection is presented in Figure 7. When the injection is started by pressing the push button, then the electric motor may rotate a certain number of revolutions to compensate for tolerances and elasticity within the conveying mechanism. The number of revolutions is counted and remains below the stored priming value defining the priming zone for the thrust range. Once non-rotation is detected by the detector, then the target speed is first reduced to zero before being automatically set at the reduced target speed. A transition time, which may be below one minute, preferably below 30 seconds is required where there is no rotation of the electric motor to relax stresses that have been initially built up in the drive train. After the transition time, the target rotational speed for the electric motor and therewith the target linear speed for the piston rod is set at the reduced speed. Once the final position for the piston rod has been reached, non-rotation of the elector motor will be detected in the final zone and the rotational speed is permanently reduced to zero. Optionally a signal is transmitted to the user after completion the cold injection.

Another example for cold injection including occlusion detection is presented in Figure 8. The detector is activated within the priming zone and the target speed is decreased to the reduced speed after the transition time. The medicament is delivered at the reduced speed and within the occlusion zone, which is in a region of the thrust range between the priming zone and the final zone, the detector is activated again as the piston rod or plunger cannot be advanced due to an occlusion within the fluid path. The control unit sets the speed, in this case the reduced target speed, permanently to zero and optionally activates an alarm signal. During the transition time for stress relief, the speed is temporarily decreased to zero. The occlusion detection also operates when the medicament is delivered at the target speed (not shown in a schematic representation). Optionally, an acoustic and/or optical alarm signal is transmitted to the user.

The conveying device for the medicament delivery device may also include the pausing button 8 which may be pressed during delivery at the target speed or at the reduced speed (Figures 9 and 10, indicated "P"). The pausing button 8 may be pressed in case the patient feels discomfort during delivery. Discomfort may be due to cold injection as the injected fluid has a lower temperature and higher viscosity as shown in Figure 10. Alternatively the active ingredient is delivered at the target speed, but the active ingredient itself may lead to discomfort such as headaches (Figure 9). The pausing button may be repeatedly pressed. The pausing button in this example is a separate button, alternatively the start button 3 switches to a pausing button if pressed for a prolonged time, for example 4 seconds.

<div align="center">

PART ANNOTATION

</div>

| 1 | Wearable bolus injector | 21 | Threaded rod |
|---|---|---|---|
| 2 | Housing | 22 | First segment |

(continued)

| | | | |
|---|---|---|---|
| 3 | Start button | 23 | Last segment |
| 4 | Release liner | 24 | Gearing |
| 5 | Adhesive patch | 25 | Cam shaft |
| 6 | Viewing window | 26 | Skin insertion mechanism |
| 7 | Status indicator | 27 | Cartridge needle insertion mechanism |
| 8 | Pause button (optional) | | |
| 9 | Pull tab | 28 | Tube |
| 10 | LED Indicator | 29 | Skin needle |
| 11 | Control unit, printed circuit board | 30 | Cartridge needle |
| | | 31 | Contacts |
| 12 | LED light | 32 | Cartridge |
| 13 | Transparent section housing | 33 | Plunger |
| 14 | Piston rod | 34 | Neck |
| 15 | Battery | 35 | Septum/crimp |
| 16 | Battery holder | 36 | "P" pausing button |
| 17 | Electric motor | | |
| 18 | Needle unit | | |
| 19 | Conveying device | | |
| 20 | Segment | | |

**Claims**

1. A conveying device for a medicament delivery device comprising:

   a piston rod which can be advanced along a thrust axis from an initial position to a final position defining a thrust range,
   a detector configured to detect when the piston rod has reached the final position,
   a conveying mechanism configured to advance the piston rod along the thrust axis,
   a control unit configured to control the advancement speed of the piston rod at a target speed,
   wherein the thrust range can be divided in at least two zones:

      a final zone whereby the control unit decreases the target speed to zero once the detector detects that the piston rod has reached the final position, and
      whereby the target speed is decreased to a reduced speed if the detector is activated in a priming zone of the thrust range.

2. The conveying device according to claim 1 whereby the priming zone is defined by that less than 30% of the thrust range has been reached by the piston rod and the final zone is starting above 80% of the thrust range.

3. The conveying device according to claim 2 wherein the target speed is stepwise or continuously decreased to zero or to the reduced speed.

4. The conveying device according to claim 3 wherein the reduced speed is below 60% of the target speed.

5. The conveying device according to claim 4 wherein within the priming zone the target speed is first reduced to zero before being set at the reduced speed.

6. The conveying device according to any of the previous claims wherein the piston rod is advanced using an electric drive comprising an electric motor and wherein the detector is activated by detecting non-rotation or limited rotation of the electric motor.

7. The conveying device according to claim 6 wherein the detector is based on detecting detects rotation or non-rotation of the electric motor by measuring the steps of the electric motor, or measuring the back-emf signal, or using an encoder, or measuring the motor current.

8. The conveying device according to claim 7 wherein the control unit activates the electric motor for advancing the piston rod at the target speed by rotation of the electric motor at a target rotational speed and wherein the control unit decreases the target rotational speed to zero when the final position is detected by the detector or decreases the target rotational speed to a reduced rotational speed for advancing the plunger at the reduced speed when the detector is activated during the priming zone.

9. The conveying device according to claim 8 wherein the control unit receives information from the detector and measures and counts the number of rotations or steps of the electric motor and compares the accumulated number of rotations or steps with stored values and if the accumulated value is below a stored priming value when non-rotation is detected then the target rotational speed is decreased to the reduced rotational speed or reduced to zero before applying the reduced rotational speed and if the accumulated value is above a stored upper value then the target rotational speed is reduced to zero.

10. The conveying device according to claim 9 wherein the control unit activates a first optical and/or acoustic signal when the piston rod is advanced at the target speed and activates a second optical and/or acoustic signal different from the first signal when the piston rod is advanced at the reduced target speed and activates a third optical and/or acoustic signal different from the first and second signals when the piston rod has reached the final position.

11. The conveying device according to claim 10 wherein the target rotational speed is decreased to zero when the accumulated number of rotations is between the priming value and the upper value and the control unit activates a fourth acoustic and/or optical alarm signal indicating an occlusion occurring in an occlusion zone located between the priming zone and the final zone preventing expelling medicament from the device.

12. The conveying device according to claims 8 to 11 further comprising a pausing button which pauses medicament delivery when pressed by setting the target rotational speed of the electric motor temporarily to zero.

13. A medicament delivery device comprising the conveying device from claims 1 to 12 further comprising a reservoir containing a medicament and a plunger moveable within the reservoir by the piston rod for expelling the medicament form the delivery device.

14. A medicament delivery device according to claim 13 wherein the initial position of the piston rod is defined by a plunger position for a full reservoir and wherein the final position is defined by a plunger engaging a neck region of the reservoir preventing further plunger advancement.

15. A medicament delivery device according to claim 14 wherein the delivery device is a wearable bolus injector.

Fig. 1

Fig. 2

Fig. 3

Fig. 4

Fig. 5

Fig. 6

Fig. 7

Detector
activation

Target speed

Reduced speed

Detector
activation

Speed [mm/min] or
Delivery rate [ml/min]

Priming zone

Time [Min]

Final zone

Fig. 8

Detector
activation

Target speed

Detector
activation

Reduced speed

Speed [mm/min] or
Delivery rate [ml/min]

Priming zone

Occlusion zone

Final zone

Fig. 9

«P»    «P»      «P» «P»

Detector
activation

Speed [mm/min] or
Delivery rate [ml/min]

Priming zone

Time [Min]

Final zone

Fig. 10

Europäisches
Patentamt

European
Patent Office

Office européen
des brevets

# EUROPEAN SEARCH REPORT

Application Number

EP 22 16 3353

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | EP 3 875 127 A1 (YPSOMED AG [CH]) 8 September 2021 (2021-09-08) * paragraph [0001] - paragraph [0064]; figure 1 * | 1-15 | INV. A61M5/142 A61M5/145 A61M5/168 A61M5/31 |
| Y | WO 2011/044706 A1 (TECPHARMA LICENSING AG [CH]; BURI THOMAS [CH]; RUFER MICHAEL [CH]) 21 April 2011 (2011-04-21) * page 5, line 3 - page 5, line 11; figures 1,2,4 * | 1-15 | |
| A | EP 3 260 149 A1 (TECPHARMA LICENSING AG [CH]) 27 December 2017 (2017-12-27) * paragraph [0069] - paragraph [0069]; figures 3,4 * | 1,13 | |

TECHNICAL FIELDS
SEARCHED      (IPC)

A61M

The present search report has been drawn up for all claims

| Place of search | Date of completion of the search | Examiner |
|---|---|---|
| Munich | 26 August 2022 | Feber, Laurent |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 22 16 3353

This annex lists the patent family members relating to the patent documents cited in the above-mentioned European search report.
The members are as contained in the European Patent Office EDP file on
The European Patent Office is in no way liable for these particulars which are merely given for the purpose of information.

26-08-2022

| Patent document cited in search report | | Publication date | Patent family member(s) | | Publication date |
|---|---|---|---|---|---|
| EP 3875127 | A1 | 08-09-2021 | EP | 3875127 A1 | 08-09-2021 |
| | | | WO | 2021175668 A1 | 10-09-2021 |
| WO 2011044706 | A1 | 21-04-2011 | CH | 702075 A1 | 29-04-2011 |
| | | | CN | 102573952 A | 11-07-2012 |
| | | | EP | 2488230 A1 | 22-08-2012 |
| | | | US | 2012265127 A1 | 18-10-2012 |
| | | | WO | 2011044706 A1 | 21-04-2011 |
| EP 3260149 | A1 | 27-12-2017 | CH | 712629 A2 | 29-12-2017 |
| | | | EP | 3260149 A1 | 27-12-2017 |
| | | | WO | 2017219157 A1 | 28-12-2017 |
| | | | WO | 2017219158 A1 | 28-12-2017 |

EPO FORM P0459

For more details about this annex : see Official Journal of the European Patent Office, No. 12/82

**EP 4 249 013 A1**

## REFERENCES CITED IN THE DESCRIPTION

**Patent documents cited in the description**

- EP 3539592 A1

- EP 3539592 A